# EUROPEAN PATENT SPECIFICATION

(11) **EP 2 055 786 B1**
(45) Date of publication and mention of the grant of the patent: **26.01.2011**
(21) Application number: 09153048.5
(22) Date of filing: 20.11.2003
(51) Int. Cl.: C12Q 1/48, G01N 33/574

(54) **Phosphospecific PAK antibodies and diagnostic kits**
Phosphospezifische PAK-Antikörper und diagnostische Kits
Anticorps PAK phosphospécifiques et trousses de diagnostic associées

(30) Priority: 27.11.2002 US 429363 P
(43) Date of publication of application: 06.05.2009
(62) Divisional of application: 03796435.0
(73) Proprietor: Sugen, Inc., New York, NY 10017 (US)
(72) Inventor: Smeal, Tod R., San Diego, CA 92121 (US); Callow, Marinella G., South San Francisco, CA 94080 (US); Jallal, Bahija, Gaithersburg, MD 20878 (US)
(74) Representative: Ford, Timothy James

(56) References cited:
- WO-A-99/63073
- WO-A2-01/36602
- WO-A2-01/53793
- CALLOW M G ET AL: "REQUIREMENTS FOR PAK4 IN THE ANCHORAGE-INDEPENDENT GROWTH OF HUMAN CANCER CELL LINES" JOURNAL OF BIOLOGICAL CHEMISTRY, AMERICAN SOCIETY OF BIOLOCHEMICAL BIOLOGISTS, BIRMINGHAM,, US, vol. 277, no. 1, 19 October 2001 (2001-10-19), pages 550-558, XP002988615 ISSN: 0021-9258
- QU JIAN ET AL: "Activated PAK4 regulates cell adhesion and anchorage-independent growth" MOLECULAR AND CELLULAR BIOLOGY, vol. 21, no. 10, May 2001 (2001-05), pages 3523-3533, XP008073557 ISSN: 0270-7306
- TOCKMAN M S ET AL: "CONSIDERATIONS IN BRINGING A CANCER BIOMARKER TO CLINICAL APPLICATION" CANCER RESEARCH, vol. 52, no. 9 SUPPL, 1992, pages 2711S-2718S, XP000614974 & NCI (NATIONAL CANCER INSTITUTE) WORKSHOP ON INVESTIGATIONAL STRATEGIES FOR DETECTION AND INTERVENTIO ISSN: 0008-5472

## Description

### FIELD OF THE INVENTION

The present invention relates to the use of phosphorylated p21-activated protein kinases (PAK) as biomarkers of tumorigenesis, phosphospecific antibodies for detecting phosphorylated PAK from mammalian biopsies and screening assays for identifying compounds that modulate PAK activity. Also contemplated is a method for determining a subset of a given population that is amenable to treatment with a compound that modulates PAK activity.

### BACKGROUND OF THE INVENTION

p21-activated protein kinase (PAK) serine/threonine kinases are important effectors of Rho family GTPases and have been implicated in the regulation of cell morphology and motility, as well as in cell transformation. See Callow et al., (2002), J. Biol. Chem., Vol. 277, Issue 1, 550-558.

One member of this kinase family, P AK4, is frequently overexpressed in human tumor cell lines of various tissue origins. The amino acid sequence of PAK4 comprises an autophosphorylation site in the kinase domain of PAK4. Mutation of this serine to glutamic acid (S474E) results in constitutive activation of the kinase. Phosphospecific antibodies directed against serine 474 detect activated PAK4 on the Golgi membrane when PAK4 is co-expressed with activated Cdc42. Furthermore, expression of the active PAK4 (S474E) mutant has transforming potential, leading to anchorage-independent growth of NIH3T3 cells. A kinase-inactive PAK4 (K350A,K351A), on the other hand, efficiently blocks transformation by activated Ras and inhibits anchorage-independent growth of HCT116 colon cancer cells. Thus, PAK4 is strongly implicated in oncogenic transformation and suggest that PAK4 activity is required for Ras-driven, anchorage-independent growth. See Callow *et al. supra.*

To date, six human PAKs, have been identified. The first subfamily consists of PAK1, PAK2, and PAK3, which exhibit 80-90% sequence identity within their catalytic domains. Members of the recently identified second PAK subfamily, PAK4, PAK5, and PAK6, are also highly related to each other but show only about 40-50% identity to the kinase domains of PAKs 1-3. All PAK family members are characterized by the presence of a p21 binding domain, which binds activated Rho family GTPases (see, Manser et al.. (1994) Nature 367, 40-46; Bagrodia et al. (1995) J. Biol. Chem. 270, 22731-22737; Knaus et al., (1995) Science 269, 221-223; Martin et al., (1995) EMBO J. 14, 1970-1978; Teo et al., (1995) J. Biol. Chem. 270, 26690-26697; and Abo et al., (1998) EMBO J. 17, 6527-6540).

As effectors of Rho family GTPases, PAK kinases play an important role in the regulation of cell morphology and motility by regulating the actin cytoskeleton. *See,* Sells et al., (1997) Curr. Biol. 7, 202-210; Sells et al., (2000) J. Cell Biol. 151, 1449-1457; Haung et al., (1998) Mol. Cell. Biol. 18, 7130-7138; Zhoa et al., (1998) Mol. Cell. Biol. 18, 2153-2163; Edwards et al., (1999) Nature Cell Biol. 1, 253-259; and Kiosses et al., (1999) J. Cell Biol. 147, 831-844. For example, PAK activity has been implicated in the localized assembly at the leading edge and disassembly at the retracting edge of focal adhesions during cell motility (Sells (1997), *supra*; Frost et al., (1998) J. Biol. Chem. 273, 28191-28198; and Eby et al., (1998) Curr. Biol. 8, 967-970). During wound healing of fibroblast monolayers, activated PAK1 rapidly localizes to the leading edge of motile cells (Sells *et al.* (2000) *supra*). Furthermore, overexpression of PAK4 has been shown to induce localized actin polymerization and the formation of filipodia (Abo *et al.,* (1998) *supra*). The PAK4-dependent changes in the actin cytoskeleton are dependent on PAK4 kinase activity and Cdc42-dependent localization to the Golgi membrane. The ability of the PAKs to modulate the actin cytoskeleton is due to kinase-dependent and -independent mechanisms.

Qu at al., ((2001) Molecular and Cellular Biology 21 (10), 3523 - 3533) suggest that PAK4 is an essential mediator of anchorage-independent growth induced by Cdc42 and Dbl family exchange factors

Since PAK4 is an effector for Cdc42, it is most likely associated with chemotaxis, cell adhesion, inflammatory responses, and innate immunological activities. For instance, there is growing evidence that implicates PAK kinases in oncogenic Ras-driven, anchorage-independent growth and in the regulation of cell survival. See, for instance, Qiu et al., (1995) Nature 374, 457-459; Qiu et al., (1995) Proc. Natl. Acad. Sci. U. S. A. 92, 11781-11785; Qiu et al., (1997) Mol. Cell. Biol. 17, 3449-3458; Khosravi-Far et al., (1995) Mol. Cell. Biol. 15, 6443-6453; Prendergast et al., (1995) Oncogene 10, 2289-2296; Keely et al., (1997) Nature 390, 632-636; Tang et al., (1997) Mol. Cell. Biol. 17, 4454-4464; Tang et al., (1998) Proc. Natl. Acad. Sci 95, 5139-5144; Tang et al., (2000) J. Biol. Chem. 275, 9106-9109; Osada et al., (1997) FEBS Lett. 404, 227-233; and Lin et al. (1999) J. Biol. Chem. 274, 23633-23641.

However, there is no suggestion in the prior art to use phosphorylated PAK4 as a biomarker for tumorogenesis in biopsy samples obtained from diseased and healthy mammals. Similarly, there is no suggestion for using PAK4 phosphospecific antibodies to determine the effect of a therapeutic composition upon a mammal undergoing treatment. Likewise, the art is silent on using PAK4 phosphorylation screening assays to identify compounds that are useful as PAK activity modulators. There also is no teaching in the prior art for detecting and comparing levels of PAK phosphorylation as a means of identifying subsets of a given population that are amenable to treatment with PAK activity modulators.

### SUMMARY OF THE INVENTION

In one aspect of the present invention, a method for selecting a mammal amenable to treatment with a PAK activity modulator is provided. This method comprises (i) determining the ratio of phosphorylated PAK4 to total PAK4 protein in a test biopsy of a tissue from a candidate mammal; and (ii) comparing the ratio of (i) to the ratio of phosphorylated PAK4 to total PAK4 protein in a control biopsy that is obtained from the same tissue type as the candidate mammal's test biopsy. In a preferred embodiment, the candidate mammal is amenable to treatment with a PAK4 activity modulator if the ratio of phosphorylated PAK4 to total PAK4 protein in the test biopsy is greater than that of the control biopsy.

In one other embodiment, the control biopsy is obtained from a healthy mammal of the same species as the candidate mammal. In yet another embodiment, the candidate mammal comprises a cancer cell. In another embodiment, the candidate mammal has a tumor.

Some embodiments of the disclosure refer to the use of PAK other than PAK4. However, the claimed invention is limited to methods involving PAK4.

The disclosure also provides a method for monitoring the effect of a therapeutic composition on a mammal. In one embodiment, the method comprises (i) measuring a first PAK phosphorylation level in a first biopsy obtained from the mammal before administration of a therapeutic composition to the mammal; and (ii) measuring a second PAK phosphorylation level in a subsequent biopsy obtained from the mammal after administration of the therapeutic composition. According to the method, a lower level of PAK phosphorylation in the subsequent biopsy as compared to the first biopsy is indicative of an effect of the therapeutic composition on the mammal.

The method also contemplates in one embodiment, that either or both of the biopsies are suspected of containing cells capable of anchorage-independent cell growth. Alternatively, In one other embodiment, neither the first nor the second biopsy is suspected of containing cells capable of anchorage-independent cell growth. In a preferred embodiment, a biopsy is a tissue biopsy. In that instance, in one embodiment, the tissue is buccal mucosa tissue, skin, hair follicles, tumor tissue, or bone marrow. The tissue also may be obtained from an internal organ from the mammal to be tested. In another embodiment, the biopsy is a biological fluid. In yet another embodiment, the biological fluid is synovial fluid, whole fresh blood, peripheral blood mononuclear cells, frozen whole blood, fresh plasma, frozen plasma, urine, or saliva. In one other embodiment, the first and subsequent biopsies are taken from a tumor in the mammal.

In another embodiment, the therapeutic composition directly or indirectly modulates the phosphorylation of at least one PAK. In a preferred embodiment, the phosphorylation of any combination of PAK4, PAK5, or PAK6 is measured. In another preferred embodiment, the phosphorylation of PAK4 is measured.

With respect to timing of dosing the mammal, in one embodiment, the first level of phosphorylated PAK in the first biopsy obtained from the mammal is measured at least 1 day, at least 5 days, at least one-week, at least two-weeks, at least one-month, before the therapeutic composition is administered to the mammal.

In one embodiment, administration of the therapeutic composition comprises at least one dose of the therapeutic composition. In another embodiment, administration of the therapeutic composition comprises a regime of multiple doses of the therapeutic composition. The doses may be administered during a period of 4 hours up to about 100 days.

In another embodiment, the subsequent biopsy obtained from the mammal in the method occurs after administration of the final dose of the therapeutic composition. In yet another embodiment, multiple biopsies are obtained from the mammal during the therapeutic composition dosing regime.

According to the method, the therapeutic composition, after administration to the mammal, may bring about, or effect, a change in one or more of physiological, biochemical, genetic, cellular, or immunological traits of the mammal.

The disclosure also provides a method for selecting a mammal amenable to treatment with a PAK activity modulator. This method comprises measuring the level of phosphorylated PAK in a test biopsy obtained from a candidate mammal. In one embodiment, a level of phosphorylated PAK in the test biopsy that is greater than the level of phosphorylated PAK in a control biopsy indicates that the mammal is amenable to treatment with a PAK activity modulator.

In one embodiment, the control biopsy is obtained from the same tissue type as the candidate mammal's test biopsy. In a preferred embodiment, the control biopsy is obtained from a healthy mammal of the same species as the candidate mammal. In another embodiment, the tissue type is lymphocyte cells. In yet another embodiment, the tissue type is brain, heart, lung, breast, skin, intestinal, colon, stomach, bladder. In a further embodiment, the mammal comprises a cancer cell.

The disclosure also provides a method for selecting from a population of mammals that have cancer, a mammal that is amenable to treatment with a PAK activity modulator. This method comprises (i) determining a first level of phosphorylated PAK in a tumorogenic biopsy obtained from a tissue from a candidate mammal; (ii) determining a second level of phosphorylated PAK in a non-tumorogenic biopsy of the same tissue from the candidate mammal of (i); and (iii) comparing the first and second levels of phosphorylated PAK. In a preferred embodiment, a level of PAK phosphorylation that is greater in the first level than the second level indicates that the candidate mammal is a mammal that is amenable to treatment with a PAK activity modulator.

The disclosure also provides a method for determining the level of phosphorylated PAK in a mammalian biopsy. This method comprises (i) exposing the biopsy to a phosphospecific antibody specific for PAK; and (ii) detecting the antibody. In a preferred embodiment, the level of antibody detected correlates with the level of phosphorylated PAK in the mammalian biopsy.

The disclosure also provides a phosphospecific antibody that is raised against a peptide of PAK4 that comprises a phosphorylated serine at position 474 of the PAK4 protein sequence. In one embodiment, the PAK4 peptide comprises the amino acid sequence, KEVPRRKSLVGTPYWMAPE, which comprises a phosphorylated serine.

The disclosure also provides a method of identifying a compound that modulates PAK phosphorylation. This method comprises (i) adding a test compound to a preparation of PAK4 protein; (ii) measuring the level of phosphorylation of the PAK4 using a phosphospecific antibody directed against PAK4; and (iii) comparing the level of the treated PAK4 preparation to the phosphorylation level of an untreated PAK4 preparation. In one embodiment, a level of phosphorylation in the treated preparation that differs from the phosphorylation level of the untreated preparation indicates that the test compound is a compound that modulates PAK phosphorylation. In a preferred embodiment, the test compound decreases, inhibits, reduces, or downregulates the level of phosphorylated PAK4 in the preparation.

In one other embodiment, the PAK4 preparation comprises PAK4 protein isolated from a biopsy from a mammal. In yet another embodiment, the PAK4 preparation comprises recombinantly-produced PAK4 protein.

The disclosure also provides a method of identifying a compound that modulates PAK phosphorylation. This method comprises, (i) exposing a culture of cells to a test compound; (ii) measuring the level of phosphorylation of PAK using a phosphospecific antibody directed against PAK4; and (iii) comparing that level to the level of PAK phosphorylation in untreated cells. In one embodiment, a level of phosphorylation that is lower or higher than the untreated cells indicates that the test compound is a compound that modulates PAK phosphorylation. In a preferred embodiment, the test compound decreases, inhibits, reduces, or downregulates the level of phosphorylated PAK produced by the treated cells.

The disclosure also provides a method for selecting a mammal amenable to treatment with a PAK activity modulator. This method comprises determining whether PAK4 protein is overexpressed in a biopsy obtained from the mammal. In one embodiment, a level of PAK4 protein that is greater than the normal level of PAK4 expression in a biopsy sample of the same tissue type, indicates that the mammal is amenable to treatment with a PAK activity modulator.

The disclosure also provides another method for selecting a mammal amenable to treatment with a PAK activity modulator. This method comprises (i) measuring the level of PAK4 protein in a biopsy obtained from a first tissue of an organ of a candidate mammal; (ii) measuring the level of PAK4 protein in a biopsy obtained from a second tissue of the organ of a candidate mammal; and (iii) comparing the two levels. In one embodiment, a difference between the levels of PAK4 protein in the two biopsies indicates that the candidate mammal is amenable to treatment with a PAK activity modulator.

Another method is disclosed which is a method for selecting a mammal amenable to treatment with a PAK activity modulator that comprises (i) determining whether PAK4 mRNA is overexpressed in a biopsy from a tissue obtained from a candidate mammal. In one embodiment, a level of PAK4 mRNA that is greater than the normal level of PAK4 mRNA expression in a biopsy obtained from an equivalent tissue from a known healthy mammal indicates that the candidate mammal is amenable to treatment with a PAK activity modulator.

The following embodiments are applicable to all methods of the present invention. Thus, in one embodiment, a mammal is selected from the group consisting of a human, rat, mouse, pig, cow, goat, monkey, cat, and dog. In a preferred embodiment, the mammal is a human. In yet another embodiment, the mammal has a disease. In another embodiment, the disease is a cancer. In yet another embodiment, the cancer is selected from the group consisting of thyroid cancer, colorectal cancer, pancreatic cancer, breast cancer, parotid cancer, synovial cell cancer, prostate cancer, laryngeal cancer, testicular cancer, hepatocellular cancer, and leiomyosarcoma.

Similarly, in a preferred embodiment of methods of the present invention, the level of phosphorylated PAK is determined by using a phosphospecific antibody specific to the phosphorylated serine of PAK4. In one embodiment, the phosphospecific antibody is raised against the PAK4 peptide, RRKSLVGTPYWMAPE, which comprises a phosphorylated serine. In another embodiment, that is applicable to methods of the present invention the level of total PAK4 protein is determined using a PAK4-specific antibody. In a preferred embodiment, the PAK4-specific antibody is raised against the peptide sequence, ATTARGGPGKAGSRGRFAGHSEA.

### BRIEF DESCRIPTION OF THE DRAWINGS

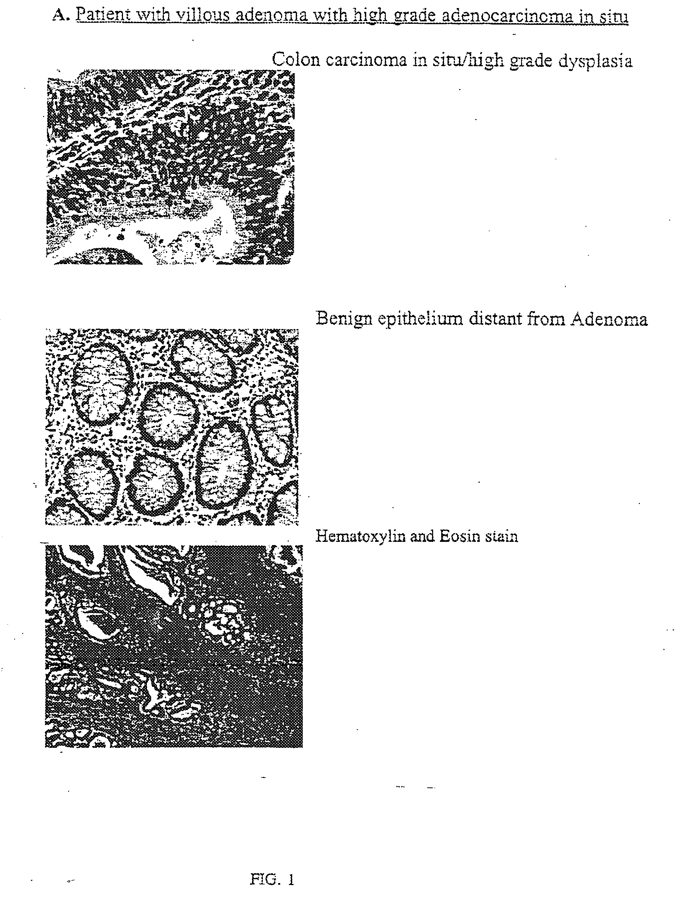

Figure 1 shows human tissue sections stained for PAK4 phosphorylation in a patient with villous adenoma with high grade adenocarcinoma in situ. Figure 1 (a) colon carcinoma in situ/high grade dysplasia; (b) benign epithelium distant from adenoma; and (c) hematoxylin and eosin stain.

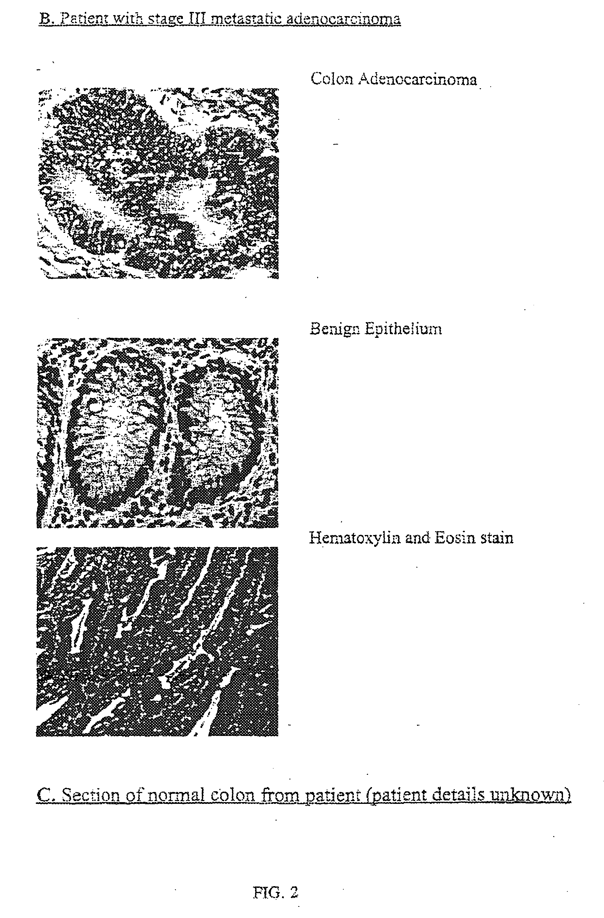

Figure 2 shows sections of a human patient stained for PAK4 phosphorylation from tissues at stage III metastatic adenocarcinoma. Figure 2 (a) colon carcinoma in situ/high grade dysplasia; (b) benign epithelium distant from adenoma; and (c) hematoxylin and eosin stain.

### DETAILED DESCRIPTION OF THE PREFERRED EMBODIMENTS

The present invention is based upon the novel use of phosphospecific PAK4 antibodies to determine levels of phosphorylated PAK in biopsies obtained from test- and control- mammalian tissues.

A "biopsy" refers to the removal and examination of a sample of tissue from a living body for diagnostic purposes.

According to the present invention, a "tissue" can be a solid tissue, such as that of any internal organ in a mammal, such as heart, lung, brain, liver, stomach, pancreas, or intestine. A "tissue" also may be exemplified by buccal mucosa tissue, skin, hair follicles, tumor tissue, or bone marrow. A tissue may also be a biological fluid, such as synovial fluid, whole fresh blood, peripheral blood mononuclear cells, frozen whole blood, fresh plasma, frozen plasma, urine, or saliva.

A "test biopsy" may be obtained from a candidate mammal, whose level of phosphorylated PAK is to be determined.

The candidate mammal can be a mammal thought to have a disease, or is known to have a disease. The disease may be cancer. For instance, the candidate mammal may be afflicted with thyroid cancer, colorectal cancer, pancreatic cancer, breast cancer, parotid cancer, synovial cell cancer, prostate cancer, laryngeal cancer, testicular cancer, hepatocellular cancer, or leiomyosarcoma. A candidate mammal also may be referred to as a "patient." Thus, a "mammal," or "individual" of the present invention, may be a human, rat, mouse, pig, cow, goat, monkey, cat, or dog.

A "control biopsy" may be obtained from a healthy mammal, such as one that is free from a disease, such as a cancer. The healthy mammal should be the same species as the candidate mammal. Accordingly, the level of PAK phosphorylation in a liver "test" biopsy from a human cancer patient is compared to the level of PAK phosphorylation in a liver "control" biopsy from a healthy human. Thus, typically, the "healthy" control biopsy is taken from the same tissue type as the tissue from which the test biopsy from the candidate mammal is taken.

Alternatively, the "control biopsy" may be taken from a non-diseased site (*e.g.*, a non-tumorogenic site) of a diseased tissue or organ from which the test biopsy is obtained. In this case, both the control and test biopsies are obtained from the same mammal. Both the control and test biopsies should be of the same size, weight, cell number, or amount, for instance, prior to determination of PAK activity. Either or both of the control and test biopsies can be modified, such as cut, diluted, or manipulated in some fashion to ensure that they are both essentially equal in cell number, size, or weight, for example.

The acronym "PAK," as used in the present invention, refers to the p21-activated protein kinases, PAK4, PAK5, and PAK6. Accordingly, being able to detect abnormally high levels of such PAKs, especially PAK4, in mammalian samples is useful for identifying compounds that modulate (*i.e.*, inhibit, decrease, downregulate, or reduce) PAK activity. Those compounds can then be formulated into therapeutic compositions and administered to mammals that require downregulation of PAK activity. Individuals that "require" downregulation of PAK activity are typically those individuals that have a population of cells that exhibit anchorage-independent growth (see below). Furthermore, detection of phosphorylated PAK protein in a sample obtained directly from an individual treated with a PAK modulator allows one to monitor the efficacy of the therapeutic treatment over time. Identifying individuals who comprise elevated levels of phosphorylated PAK protein, is a useful first step in identifying individuals who may be amenable to treatment with a PAK modulator.

To that end, an individual who has a level of phosphorylated PAK in a test biopsy that is higher than that normally associated with the tissue from which it was obtained, has an abnormal level of PAK that is potentially treatable with a PAK modulator. A "modulator" of the present invention preferably decreases PAK activity. Elevated levels of phosphorylated PAK, especially activated PAK4, correlate well with cells that have an increased propensity to undergo anchorage-independent growth. The latter phenomenon is a mechanism known to be an attributable factor in tumor formation and cancerous growths. For instance, it is known that anchorage independent growth means that viable cells obtained from a tumor biopsy can form colonies in semi-solid culture medium. Conversely, anchorage *dependent* cells cannot form such colonies. Accordingly, a level of phosphorylated PAK that is higher than that which is considered normal, is a good biomarker of tumorigenesis, because such elevated levels are indicators of anchorage independent cell growth.

The disclosed methods apply the detection of baseline, increased, and abnormal levels of PAK phosphorylation, to various diagnostic and therapeutic ends. For instance, one disclosed method monitors the effect of a therapeutic composition on a mammal, such as a human patient, by comparing the level of PAK phosphorylation in biopsy samples taken before and after treatment with the therapeutic composition. Prior to treating the individual, however, the individual preferably should first be identified as actually requiring such treatment. Accordingly, determining the activity of endogenous PAK in a patient helps to determine whether the patient's PAK activity needs to be downregulated. The present invention provides a method for selecting a mammal that is amenable to treatment with a PAK4 activity modulator.

Such a selection method can be accomplished in a variety of ways. For example, one may determine the ratio of phosphorylated PAK to total PAK4 protein in a test biopsy obtained from a tissue from a candidate mammal. Those levels can then be compared to the same ratio measured in the same tissue type, but in a healthy, control mammal of the same species as the candidate mammal. One may test a number of such healthy individuals in order to obtain a set of "normal" phosphorylated PAK to total PAK4 protein ratios for particular organs and tissues. Accordingly, a ratio, from a patient, that is greater than normal indicates that that patient is likely to be amenable to treatment with a PAK activity modulator. Alternatively, the phosphorylated PAK-to-PAK4 ratio can be compared in the test biopsy and a control biopsy from the same patient.

The patient, in this context, may already be diagnosed with a disease, like cancer. Thus, the present invention contemplates selecting a subset of cancer patients that would benefit from treatment with a PAK modulator. Accordingly, one could use the inventive method to compare the levels of phosphorylated PAK in a test biopsy of a tumor from a patient (*i.e.*, "candidate mammal") with a control biopsy obtained from a non-tumorogenic tissue from the same, or adjacent organ, or tissue. In keeping with the invention, a higher level of PAK phosphorylation in the tumor biopsy than in the non-tumorogenic tissue indicates that that cancer patient is amenable to treatment with a PAK activity modulator.

According to the present invention, phosphorylated PAK4, and total PAK4 protein, can be detected using antibodies that recognize the phosphorylated form of PAK4 and the PAK4 protein in general. The present invention provides a method, then, for determining the level of phosphorylated PAK in a mammalian test biopsy by exposing the test biopsy to, for instance, a phosphospecific antibody specific for PAK. The antibody can be labelled so that it is easily detectable. Thus, the amount of antibody that binds to phosphorylated PAK4 in a sample, correlates with the level of phosphorylated PAK in the test biopsy. Such a phosphospecific antibody can be designed to the phosphorylated serine-474 of the PAK4 protein. For instance, an antibody can be raised against the PAK4 peptide sequence, RRKSLVGTPYWMAPE (SEQ ID NO. 1), wherein the serine-474 comprises a phosphate moiety (bold, underlined). Antibodies raised to other peptide sequences that comprise the phosphorylated serine-474 are also contemplated by the present invention.

Similarly, the peptide sequence, ATTARGGPGKAGSRGRFAGHSEA (SEQ ID NO. 2), is unique to PAK4 and can be used to produce an antibody that recognizes and binds to this particular sequence. Accordingly, an antibody raised against SEQ ID NO. 2 for detecting PAK4 protein *per se* is contemplated by the present invention.

Antibodies of the present invention may be monoclonal, polyclonal, or a specific recombinant single chain molecule. An antibody of the present invention also may be labeled so that it is detectable. For instance, an antibody can be conjugated or crosslinked to an enzyme, fluorophore, or chromophore to aid in detection of the antibody once it is bound to its intended target. Alternatively, one may use a similarly labeled secondary antibody which binds to the PAK-specific antibody, thereby facilitating detection of the bound antibody.

With such antibodies, it is possible to screen for test compounds that modulate PAK activity. The disclosure also provides a method of identifying a compound that modulates PAK phosphorylation by measuring the level of phosphorylation of isolated and/or purified PAK4 before and after administering a test compound. The isolated or purified PAK4 can be obtained from lysed cells or produced recombinantly. The test compound also could be administered to whole cells which are subsequently tested with a phosphospecific PAK antibody to determine the effect of the test compound on PAK activity in the cells. Once identified as modulating PAK activity, the test compound may be formulated into a therapeutic composition for administering to a mammal *in vivo, ex vivo* and *in vitro.*

Prior to treatment with a therapeutic composition, a test biopsy may be taken from the targeted mammal to establish the level of PAK phosphorylation in a particular tissue. Thus, the test biopsy may be taken 1, 5, 7, 14, or 30 days, for instance, prior to treatment with the therapeutic composition. Multiple test biopsies may be taken from the same tissue during that time to determine an "average" PAK phosphorylation level and to account for possible fluctuations in those levels. After such time, the mammal may then be placed on a particular dosing regime whereupon the therapeutic composition is administered daily, weekly, or monthly, for instance. The therapeutic composition may be administered in at least one dose of the therapeutic composition, or at least two doses, or at least 5 doses or at least 10 doses, up to at least 55 or 56 doses. These doses may be administered during a period of 4 hours up to about 100 days. The doses can be administered over a period of 24 hours, 2 days, or 28 days. Alternatively, two doses can be administered per every 24 hours or administered about every 12 hours. It will be understood by those of skill in the art that the administration of the therapeutic composition can be varied to suit individual needs of the mammal being treated. For example, in a typical dosing regimen, the patient receives two doses per day of therapeutic composition, for a number of days, such as about 28 or about 56 days. In other dosing regimens, the therapeutic composition is administered about once per day, twice per week, or once per week.

After the dosing regiment has ended, another, subsequent biopsy is taken from the same tissue as that used to provide the "test" biopsy. The level of PAK phosphorylation in this subsequent biopsy is then determined according to the inventive methods and compared with those levels determined prior to treatment The effect of the therapeutic composition on the mammal can therefore be identified by a decrease in the level of PAK phosphorylation after treatment, or by identifying changes in physiological, biochemical, genetic, or immunological aspects of the mammal. Biopsies also may be taken during the dosing regime and used to determine the efficacy of the therapeutic composition on the mammal while the treatment is on-going.

The examples below are intended to illustrate but not limit the invention. While they are typical of those that might be used, other procedures known to those skilled in the art may be used.

### EXAMPLES

### EXAMPLE I

### ANTIBODIES

Rabbit polyclonal antibodies against total PAK4 protein (#933) were raised against peptide ATTARGGPGKAGSRGRFAGHSEA (SEQ ID NO. 2), which represents amino acids 122-144 of the PAK4 protein. Phosphospecific anti-PAK4 polyclonal rabbit antibody #108 was raised against the KLH conjugated phospho-peptide synthesized with Ser-474 phosphorylated (CRRKpSLVGTPYWMAPE) (SEQ ID NO. 1). The seqeunce, RRKSLVGTPYWMAPE, of SEQ ID NO. 1 spans the region 371-385 of the PAK4 protein sequence. The phosphospecific PAK4 polyclonal #108 sera was further purified on a protein A affinity column. The specificity of the protein A purified sera for phosphorylated PAK4 was confirmed by testing against antigen peptides CRRKpSLVGTPYWMAPE (SEQ ID NO. 1), the non-phosphorylated CRRKSLVGTPYWMAPE (SEQ ID NO. 3) (*i.e.*, peptide "#704") and phosphorylated Thr-478 (CRRKSLVGpTPYWMAPE) (SEQ ID NO. 4) (*i.e.,* peptide "#681") versions of the PAK4 peptide to confirm specificity to the phosphorylated serine 474. Any antibodies that were found to cross-react with the non-phosphorylated peptide were removed by passing the protein A purified sera through peptide #681 coupled to Ultralink-Iodoacetyl column (Pierce, USA).

For Western blot analysis, the protein A purified phosphospecific anti-PAK4 (phosphorylated serine 474) sera was used at 1/4000 dilution. For immunofluorescence experiments, the protein A purified phosphospecific anti-PAK4 antibody was pre-abdorbed with peptides #681 and #704. Dilutions of the PAK4 antibodies used for immunohistochemistry are as follows: For #108 dilution range of 1/250 to 1/100 was preferred for IHC methods; for #933 dilution range varied 1/50 for the vector red method, while a dilution as high as 1/200 was used for the ABC staining method.

### Example II

### IHC Protocol for Rabbit Primary Antibodies

A microtome was used to cut tissue sections at 4-5 microns. See Table 1 below for examples of tissue types used in the present invention. The sections were floated on a water bath and picked up onto slides. Slides containing paraffin sections were deparaffinized using xylene and alcohol, rehydrated, and then subjected to the steam method of target retrieval (DAKO reagent #S1700). Immunohistochemical experiments were performed on a DAKO autostainer following the procedures and reagents developed by DAKO. Specifically, the slides were blocked (the blocking agent was included with the Vectastain ABC-AP kit), rinsed, primary antibody applied, incubated for 45 minutes at room temperature, and the slides rinsed again. Biotinylated secondary antibody (Vector anti-rabbit secondary antibody, BA-100, diluted at 5µl/ml) was then applied to the tissue on the slide. The slides were then incubated for 30 minutes at room temperature, rinsed, and Vector ABC-AP (AK-5000) reagent applied. Vector Red (SK-5100) was used as a substrate for Vector ABC-AP (AK-5000). The control antibody, CD31, was obtained from DAKO (M0823 mouse monoclonal) and used following their conditions at a 1:80 dilution to ensure the quality and integrity of the sample tissue.

### Example III

### ABC Immunoperoxidase Procedure

### (i) Slide preparation

1. Cut 4µm tissue sections onto charged slides. See Table 1 below for examples of tissue types used in the present invention. Place slides in a 60°C drying oven for 20 minutes. Alternatively, the slides can be air dried overnight, if desired.
2. Label slides and place in racks.
3. Deparaffinize and rehydrate the slides (3 changes of xylene—10 minutes followed by 3X 100% ethanol, 2X 95% ethanol—total 10 minutes; rinse with distilled water).
4. Block endogenous peroxidase: Place slides in a 3% hydrogen peroxide for 5 minutes.
5. Rinse slides in distilled water.
6. Pretreatments: Perform pretreatment if desired. Note that possible pretreatment includes protease or other enzyme digestion of heating of slides in 10 mM citrate buffer pH 6.0 or EDTA buffer, pH 8.0. Allow heat treated slides to cool gradually (for 20 minutes in the buffer) before transfer to distilled water. Do not let the slides dry out.
7. Endogenous Biotin Block: Perform biotin blocking procedures if desired, using, for instance a Vector of DAKO kit. Alternatively, use the following method: add 200 ml of distilled water to 75 ml of egg substitute; soak slides for 15 minutes in the egg mix, rinse in distilled water two times; soak the slides for 15 minutes in skim milk; rinse in distilled water two times; transfer the slides to PBST (PBS buffer plus Tween).
8. Wash in three changes of PBST; however, note that some antibodies are Tween-20 sensitive, in which case Tween-20 should not be added to the PBS buffer.

### (ii) Antibody Application

1. Primary: Dilute mouse primary antibody (*i.e.*, either the phosphospecific PAK4 antibody or the totak PAK4 antibody) in PBS with 1% BSA. For purified monoclonal antibodies, concentrations in the range of 1-5µg/ml arc usually optimum. Actual concentrations must be determined, however, by titration. Apply an adequate volume, for instance, about 100 µl, of the diluted primary antibody to the tissue; cover the entire tissue.
2. Incubate the slides for 40 minutes in a moist chamber at room temperature, or overnight at 4°C. Meanwhile prepare secondary antibody and tertiary reagent (for instance, Vector biotinylated horse anti-mouse IgG (H+L) diluted 1:300 for monoclonal mouse primaries and Vector Elite ABC). Typically, the secondary antibody targets the same species in which the primary antibody was made.
3. Wash slides in three changes of PBST solution.
4. Apply the biotinylated secondary antibody and incubate for 30 minutes in a moist chamber at room temperature.
5. Wash slides in 3 changes ofpBST as above.
6. Apply tertiary (ABC) to slides and incubate for 25 minutes in a moist chamber at room temperature.
7. During incubation fill two slide rack containers with 175 ml of 0.05M Tris buffer and place in a 37°C waterbath to prewarm. Thaw a frozen aliquot of diaminobenzidine (DAB).
8. Wash in 3 changes of PBST for a minimum of 10 minutes.
9. Rinse slides in prewarmed 0.05M Tris buffer, pH 7.8-8.0.
10. Place slides in a solution of DAB and incubate for 8 minutes.
11. Wash the slides two times in distilled water.
12. Counterstain the slides in Mayer's hematoxylin for 25-30 seconds.
13. Rinse slides in water until clear.
14. Place slides in blue hematoxylin, that is dissolved 0.5% ammonia water, for about 30 seconds. Wash with three changes of water.
15. Dehydrate the slides 95% ethanol twice and with 100% ethanol three times, and clear with three washes of xylene.
16. Coverslip using xylene compatible mounting medium and glass coverslip or tape.

### Example IV

### Immunofluorescence

Coverslips were placed in 24-well tissue culture trays and seeded at a density of 2 x 10⁴ cells per well. Cells were transfected the following day with a mammalian expression vector that drives expression of a human PAK4 clone. The medium was replaced six hours post transfection and the cells were alllowed to grow for 48 hours. Coverslips were washed extensively with PBS and then fixed with cold 4% paraformaldehyde. The fixed cells were solubilized using a standard solution of 0.5% Triton-X-100, washed and incubated with primary antibodies diluted in PBS containing 1% BSA, Protein A purified rabbit serum directed against peptide #682 (phosphospecific-S474), desalted and specificity for phosphospecific-S474 confirmed by Western blot analysis prior to use. HA-7 antibody (Sigma, USA) was used to detect exogenous PAK4. The expressed PAK4 comprises an HA-7 antibody-recognized epitope tag fused to its N-terminal. The coverslips were washed extensively with PBS and incubated with a rhodamine-conjugated goat α-rabbit secondary antibody (Santa Cruz Biotechnology, Inc., USA) or with a FITC-conjugated goat α-mouse-fluoroscein isothiocyanate ("FITC") antibody (Santa Cruz Biotechnology, Inc., USA), together with 1 µg/ml of Hoechst No. 33342 (bis-Benzimide, Sigma, USA) in the dark for two hours at room temperature. The coverslips were washed and mounted in Fluorosave (Calbiochem Corp., USA) and allowed to dry overnight. Images were acquired using a Nikon oil emersion 40 x/1.30 objective on a Nikon Eclipse E800 microscope with a SPOT RT camera.

**Table 1**

| | **Sample** | **Tissue** | **Diagnosis** | **Age/Sex** |
|---|---|---|---|---|
| 1 | 1 | Brain | Normal | 74 M |
| | 2 | Brain | Normal | N/A |
| 2 | 1 | Brain | Glioblastoma Multiforme | 49 M |
| | 2 | Brain | Glioblastoma Multiforme | 49 M |
| 3 | 1 | Breast | Normal | 49 F |
| | 2 | Breast | Normal | 49 F |
| 4 | 1 | Breast | Adenocarcinoma. Metastatic | 79 F |
| | 2 | Breast | Adenocarcinoma, Metastatic | 62 F |
| 5 | 1 | Breast | Adenocarcinoma, Intraductal | F |
| | 2 | Breast | Adenocarcinoma, Intraductal | 54 F |
| 6 | 1 | Breast | Adenocarcinoma, Non-Metastatic Local | F |
| | 2 | Breast | Adenocarcinoma, Non-Metastatic Local | Addendum |
| 7 | 1 | Colon | Normal | N/A |
| | 2 | Colon | Normal | F |
| 8 | 1 | Colon | Adenocarcinoma, In Situ or Microinvasive | N/A |
| | 2 | Colon | Adenocarcinoma, In Situ or Microinvasive | 68 M |
| 9 | 1 | Colon | Adenocarcinoma, Metastatic | 54 M |
| | 2 | Colon | Adenocarcinoma, Metastatic | 57 F |
| 10 | 1 | Colon | Adenocarcinoma, Non-Metastatic Local | 68 M |
| | 2 | Colon | Adenocarcinoma, Non-Metastatic Local | 75 F |
| 11 | 1 | Kidney | Normal | 52 F |
| | 2 | Kidney | Normal | 49 F |
| 12 | 1 | Kidney | Renal cell Carcinoma <7cm | 66 M |
| | 2 | Kidney | Renal cell Carcinoma <7cm | 33 M |
| 13 | 1 | Kidney | Renal cell Carcinoma >7cm | 67 M |
| | 2 | Kidney | Renal cell Carcinoma >7cm | 79 M |
| 14 | 1 | Lung | Normal | 36 F |
| | 2 | Lung | Normal | 76 F |
| 15 | 1 | Lung | Adenocarcinoma, Metastatic | 65 M |
| | 2 | Lung | Adenocarcinoma, Metastatic | 34 F |
| 16 | 1 | Lung | Adenocarcinoma, Non-Metastatic (T1, Stage fa) | 75 F |
| | 2 | Lung | Adenocarcinoma, Non-Metastatic (T1, Stage Ia) | 66 M |
| 17 | 1 | Lung | Adenocarcinoma, Non-Metastatic (T2, Stage Ib) | 73 M |
| | 2 | Lung | Adenocarcinoma, Non-Metastatic (T2, Stage Ib) | Addendum |
| 18 | 1 | Ovary | Normal | F |
| | 2 | Ovary | Normal | F |
| 19 | 1 | Ovary | Borderline Serous or Mucous Tumor | F |
| | 2 | Ovary | Borderline Serous or Mucous Tumor | 26 F |
| 20 | 1 | Ovary | Papillary Serous Carcinoma | 39 F |
| | 2 | Ovary | Papillary Serous Carcinoma (Limited to Ovary) | Addendum |
| | 3 | Ovary | Papillary Serous Carcinoma (Limited to Ovary) | Addendum |
| 21 | 1 | Ovary | Papillary Serous Carcinoma with Metastasis | 26 F |
| | 2 | Ovary | Papillary Serous Carcinoma with Metastasis | F |
| 22 | 1 | Prostate | Normal | 18 M |
| | 2 | Prostate | Normal | 55 M |
| 23 | 1 | Prostate | Adenocarcinoma (Gleason 2-6) | 64 M |
| | 2 | Prostate | Adenocarcinoma (Gleason 2-6) | 65 M |
| 24 | 1 | Prostate | Adenocaroinoma (Gleason 7-10) | 66 M |
| | 2 | Prostate | Adenocarcinoma (Gleason 7-10) | 60 M |
| 25 | 1 | Prostate | Adenocarcinoma, Metastatic | 75 M |
| | 2 | Prostate | Adenocarcinoma, Metastatic | 71 M |
| 26 | 1 | Skin | Normal | 72 M |
| | 2 | Skin | Normal | 7 F |
| 27 | 1 | Skin | Angiosarcoma | 92 F |
| | 2 | Skin | Angiosarcoma | Addendum |
| 28 | 1 | Skin | Kaposi's Sarcoma | 46 M |
| | 2 | Skin | Kaposi's Sarcoma | Addendum |
| 29 | 1 | Vessel, Artery, Coronary | Normal | 69 M |
| | 2 | Vessel, Artery, Coronary | Normal | 26 F |

### Example V

### Results

The data for the phosphospecific antibody (#108) in colon carcinomas is especially informative (6 out of 6 patients showed marked perinuclear staining in tumor and not distal benign tissue; note that staining was detected using vector red substrate, which gives a fuchsia/red-colored deposit. This result strongly suggests that PAK4 is specifically active in the colon tumor cells and not in benign colon tissue from the same patient. Staining of phosphorylated PAK4 was also observed in renal cell carcinoma, lung adenocarcinoma, prostatic adenocarcinoma, intraductal breast adenocarcinoma, and ovarian adenocarcinoma.

In tumors, strong staining with phosphospecific-PAK4 antibody was identified in colonic adenocarcinomas (while distal benign tissue failed to show phospho-PAK4 staining). On a scale of 0-3, "0" indicates no staining, "1" is indicative of weak staining, "2" indicates moderate staining and "3" indicates strong staining. Adenomatous epithelium was faintly to moderately positive, but most normal epithelium showed only staining of "1" for phosphorylated PAK4. Prostatic adenocarcinomas showed moderate staining ("2").

In benign tissues, the most prominent staining for phosphorylated PAK4 was seen in adipocytes, cardiac myocytes, sebaceous glands, and occasional macrophages. Additional positive cell and tissue types included hair follicles, benign prostatic epithelium, breast epithelium, and urothelium. Negative cell types for phosphorylated PAK4 included pneumocytes, oocytes, fibroblasts, glial cells in the brain, cortical neurons, renal glomeruli, renal tubular epithelium other than collecting ducts, oocytes, ovarian stroma, and ovarian surface epithelium.

Occasionally, neutrophils and eosinophils were positive. Less prominent positivity was identified in renal collecting ducts, thick loops of Henle, mast cells, and benign colonic epithelium. Weak staining ("1") was identified in respiratory epithelium, ovarian granulosa-cells, theca cells, eccrine sweat glands, prostatic stroma, and focally in endothelium and vascular smooth muscle. Several samples of squamous epithelium were stained in the basal layer. Lymphocytes also showed occasional staining.

Accordingly, a level of phosphorylated PAK4 that is above normal in a certain tissue is a useful biomarker for determining the integrity and status of the cells in the tissue.

## Claims

1. A method for selecting a mammal amenable to treatment with a PAK4 activity modulator, comprising:
(i) determining the ratio of phosphorylated PAK4 to total PAK4 protein in a test biopsy of a tissue from a candidate mammal; and
(ii) comparing the ratio of (i) to the ratio of phosphorylated PAK4 to total PAK4 protein in a control biopsy that is obtained from the same tissue type as the candidate mammal's test biopsy,
wherein the candidate mammal is amenable to treatment with a PAK4 activity modulator if the ratio of phosphorylated PAK4 to total PAK4 protein in the test biopsy is greater than that of the control biopsy.

2. The method of claim 1, wherein the control biopsy is obtained from a healthy mammal of the same species as the candidate mammal.

3. The method of claim 1, wherein the candidate mammal comprises a cancer cell.

4. The method of claim 1, wherein the candidate mammal has a tumor.

5. The method of any one of the preceding claims, wherein the candidate mammal is selected from the group consisting of a human, rat, mouse, pig, cow, goat, monkey, cat, and dog.

6. The method of claim 5, wherein the mammal is a human.

7. The method of any one of the preceding claims, wherein the level of phosphorylated PAK4 is determined by using a phosphospecific antibody specific to the phophorylated serine of PAK4.

8. The method of claim 7, wherein the phosphospecific antibody is raised against the PAK4 peptide, RRKSLVGTPYWMAPE (SEQ ID NO: 1), which comprises a phosphorylated serine.

9. The method of any one of the preceding claims, wherein the level of total PAK4 protein is determined using a PAK4-specific antibody.

10. The method of claim 9, wherein the PAK4-specific antibody is raised against the peptide sequence ATTARGGPGKAGSRGRFAGHSEA (SEQ ID NO: 2).

## Patentansprüche

1. Verfahren zum Auswählen eines Säugers, der für eine Behandlung mit einem Modulator der PAK4-Aktivität zugänglich ist, umfassend:
(i) Bestimmen des Verhältnisses von phosphorylierter PAK4 zu Gesamt-PAK4-Protein in einer Testbiopsie eines Gewebes aus einem Kandidatensäuger und
(ii) Vergleichen des Verhältnisses von (i) mit dem Verhältnis von phosphorylierter PAK4 zu Gesamt-PAK4-Protein in einer Kontrollbiopsie, die aus demselben Gewebetyp wie die Testbiopsie des Kandidatensäugers erhalten wird,
wobei der Kandidatensäuger für eine Behandlung mit einem Modulator der PAK-4-Aktivität zugänglich ist, wenn das Verhältnis von phosphorylierter PAK4 zu Gesamt-PAK4-Protein in der Testbiopsie größer als das der Kontrollbiopsie ist.

2. Verfahren nach Anspruch 1, wobei die Kontrollbiopsie von einem gesunden Säuger derselben Spezies wie der Kandidatensäuger erhalten wird.

3. Verfahren nach Anspruch 1, wobei der Kandidatensäuger eine Krebszelle umfasst.

4. Verfahren nach Anspruch 1, wobei der Kandidatensäuger einen Tumor hat.

5. Verfahren nach einem der vorangehenden Ansprüche, wobei der Kandidatensäuger aus der Gruppe, bestehend aus einem Menschen, einer Ratte, einer Maus, einem Meerschweinchen, einer Kuh, einer Ziege, einem Affen, einer Katze und einem Hund, ausgewählt wird.

6. Verfahren nach Anspruch 5, wobei der Säuger ein Mensch ist.

7. Verfahren nach einem der vorangehenden Ansprüche, wobei die Konzentration an phosphorylierter PAK4 unter Verwendung eines Phosphor-spezifischen Antikörpers, der für das phosphorylierte Serin von PAK4 spezifisch ist, bestimmt wird.

8. Verfahren nach Anspruch 7, wobei der Phosphor-spezifische Antikörper gegen das PAK4-Peptid, RRKSLVGTPYWMAPE (SEQ ID NO: 1), welche ein phosphoryliertes Serin umfasst, gerichtet ist.

9. Verfahren nach einem der vorangehenden Ansprüche, wobei die Konzentration an Gesamt-PAK4-Protein unter Verwendung eines PAK4-spezifischen Antikörpers bestimmt wird.

10. Verfahren nach Anspruch 9, wobei der PAK4-spezifische Antikörper gegen die Peptidsequez ATTARGGPGKAGSRGRFAGHSEA (SEQ ID NO: 2) gerichtet ist.

## Revendications

1. Procédé de sélection d'un mammifère pouvant être sensible à un traitement par un modulateur d'activité PAK4, comprenant :
(i) la détermination du rapport entre la protéine PAK4 phosphorylée et la protéine PAK4 totale dans une biopsie d'essai d'un tissu provenant du candidat mammifère ; et
(ii) la comparaison du rapport issu de (i) au rapport entre la protéine PAK4 phosphorylée et la protéine PAK4 totale dans une biopsie témoin qui est obtenue à partir du même type de tissu que la biopsie d'essai du candidat mammifère,
procédé dans lequel le candidat mammifère peut être sensible à un traitement par un modulateur d'activité PAK4 si le rapport entre la protéine PAK4 phosphorylée et la protéine PAK4 totale dans la biopsie d'essai est supérieur à celui de la biopsie témoin.

2. Procédé selon la revendication 1, dans lequel la biopsie témoin est obtenue à partir d'un mammifère sain de la même espèce que le candidat mammifère.

3. Procédé selon la revendication 1, dans lequel le candidat mammifère comprend une cellule cancéreuse.

4. Procédé selon la revendication 1, dans lequel le candidat mammifère a une tumeur.

5. Procédé selon l'une quelconque des revendications précédentes, dans lequel le candidat mammifère est sélectionné dans le groupe consistant en un être humain, un rat, une souris, un porc, une vache, une chèvre, un singe, un chat et un chien.

6. Procédé selon la revendication 5, dans lequel le mammifère est un être humain.

7. Procédé selon l'une quelconque des revendications précédentes, dans lequel le niveau de PAK4 phosphorylée est déterminé en utilisant un anticorps phosphospécifique, spécifique à la sérine phosphorylée du PAK4.

8. Procédé selon la revendication 7, dans lequel l'anticorps phosphospécifique est dirigé contre le peptide PAK4, RRKSLVGTPYWMAPE (SEQ ID NO : 1) qui comprend une sérine phosphorylée.

9. Procédé selon l'une quelconque des revendications précédentes, dans lequel le niveau de la protéine PAK4 totale est déterminé en utilisant un anticorps spécifique à la PAK4.

10. Procédé selon la revendication 9, dans lequel l'anticorps spécifique à la PAK4 est dirigé contre la séquence peptidique ATTARGGPGKAGSRGRFAGHSEA (SEQ ID NO : 2).


### SEQUENCE LISTING

<110> Sugen, Inc.
<120> Phosphospecific PAK antibodies and diagnostic kits
<130> PC23574
<150> us 60/429,363
   <151> 2002-11-27
<160> 5
<170> PatentIn version 3.3
<210> 1
   <211> 16
   <212> PRT
   <213> Homo sapiens
<220>
   <221> MOD_RES
   <222> (5)..(5)
   <223> Phosphorylated Ser
<400> 1
   Cys Arg Arg Lys Ser Leu Val Gly Thr Pro Tyr Trp Met Ala Pro Glu
   1 5 10 15
<210> 2
   <211> 23
   <212> PRT
   <213> Homo sapiens
<400> 2
   Ala Thr Thr Ala Arg Gly Gly Pro Gly Lys Ala Gly Ser Arg Gly Arg
   1 5 10 15
   Phe Ala Gly His Ser Glu Ala
   20
<210> 3
   <211> 16
   <212> PRT
   <213> Homo sapiens
<400> 3
   Cys Arg Arg Lys Ser Leu Val Gly Thr Pro Tyr Trp Met Ala Pro Glu
   1 5 10 15
<210> 4
   <211> 16
   <212> PRT
   <213> Homo sapiens
<220>
   <221> MOD_RES
   <222> (9)..(9)
   <223> Phosphorylated Thr
<400> 4
   Cys Arg Arg Lys Ser Leu Val Gly Thr Pro Tyr Trp Met Ala Pro Glu
   1 5 10 15
<210> 5
   <211> 19
   <212> PRT
   <213> Homo sapiens
<220>
   <221> MOD_RES
   <222> (8)..(8)
   <223> Phosphorylated Ser
<400> 5
   Lys Glu Val Pro Arg Arg Lys Ser Leu Val Gly Thr Pro Tyr Trp Met
   1               5                   10                  15
   Ala Pro Glu